# EUROPEAN PATENT SPECIFICATION

(11) **EP 4 381 979 B1**
(45) Date of publication and mention of the grant of the patent: **09.09.2026**
(21) Application number: 22852384.1
(22) Date of filing: 03.08.2022
(51) Int. Cl.: A41D 13/11, A61L 2/10, A61F 9/04

(54) **LOADING BASE FOR FACIAL PROTECTION DEVICES**
LADEBODEN FÜR GESICHTSSCHUTZVORRICHTUNGEN
BASE DE CHARGE POUR DISPOSITIFS DE PROTECTION FACIALE

(30) Priority: 04.08.2021 ES 202131613 U
(43) Date of publication of application: 12.06.2024
(73) Proprietor: Morales, Alvarez Jose Carlos, 30394 Las Lomas de Canteras, (Cartagena) Murcia (ES)
(72) Inventor: Morales, Alvarez Jose Carlos, 30394 Las Lomas de Canteras, (Cartagena) Murcia (ES)
(74) Representative: Ibarra Garcia, Isabel
(86) International application number: PCT/ES2022/000030
(87) International publication number: WO 2023/012387

(56) References cited:
- CN-A- 103 025 444
- CN-U- 203 954 237
- ES-U- 1 253 694
- ES-U- 1 253 694
- ES-U- 1 253 694
- JP-U- 3 211 531
- KR-A- 20210 136 887
- KR-A- 20210 136 887
- US-A1- 2021 016 332

## Description

### FIELD OF THE ART

The present invention relates to a loading base for facial protection devices of the type used in healthcare settings by medical personnel, nurses, assistants, dentists, etc., and materialized as facial protection equipment which is equipped with various elements that facilitate healthcare practice, such as a LED lamp, a camera, a microphone, and swinging elements to facilitate moving the visor with respect to the support forming the device.

The object of the invention is to provide a base which, in addition to allowing a base for supporting or holding the work screen, allows recharging different electronic equipment associated with the facial protection device, and allows disinfecting both the outer face and the inner face of the transparent glass or plastic protective screen or visor of said device.

### BACKGROUND OF THE INVENTION

Facial protection devices used in healthcare settings by medical personnel, nurses, assistants, dentists, and the like, and materialized as facial protection equipment which is equipped with various electronic elements that facilitate healthcare practice, such as a LED lamp, a camera, a microphone, and swinging elements to facilitate moving the visor with respect to the support forming the device, are known.

A device of this type is described in utility model U202031414, such that said device further includes in its housing a series of UV LEDs which project lights beans towards the outer surface of the screen.

This solution is not satisfactory because the UV light is projected exclusively on the outer face of the screen, to which it should be further mentioned that the dimensions of said screen are often greater than the effective reach of the UV LEDs.

At the same time, it is known that this type of lighting poses a risk to the user's eyes, so it is preferable for the disinfection means of the device to be independent of said device in order to ensure that said disinfection operations cannot be performed with the device applied on the user's head. ES1253694U discloses a loading base for facial protection devices, being of the type of bases that are made up of a main housing having support arms on which a facial protection device with a screen rests.

### DESCRIPTION OF THE INVENTION

The proposed loading base for facial protection devices solves in a fully satisfactory manner the problems set forth above by ensuring perfect disinfection of the facial protection device in a completely safe manner.

To that end, the loading base for facial protection devices is made up of a main housing having support arms on which a facial protection device with a screen rests.

Based on this conventional structuring, the base has been envisaged to incorporate a bridge that can move vertically on the support arms which, in an inoperative situation, is flush with the main housing of the base, wherein the bridge has a structure in which two parallel arches are defined determining an arched slot, according to the curvature and horizontal section of the screen of the facial protection device, said bridge incorporating inside its arches a plurality of UV LEDs for disinfecting both surfaces of the screen of the facial protection device.

To that end, the mentioned bridge can preferably move vertically from a pair of vertical augers associated with the support arms determining the support on which the facial protection device rests, these augers rotate in a synchronized manner by being linked to the ends of the bridge through the corresponding threading, being actuated through a transmission linked to an electric motor.

Specifically, the ends of the bridge incorporate perforations through which the bridge is linked to the vertical augers, the rotation of the vertical augers being actuated by means of a transmission linked to an electric motor.

In this way, the bridge can move vertically and in a controlled manner along the entire surface of the visor of the facial protection device, applying UV light at a minimum distance from said screen, which ensures perfect disinfection of the visor on both faces, such that once the disinfection process ends, the arch will return to its retracted position being flush with the main housing of the base.

As an additional safety measure, the base has been envisaged to include proximity sensors which detect the presence of any people in the vicinities of the base and through which said disinfection process is stopped momentarily until the area is clear.

### BRIEF DESCRIPTION OF THE DRAWINGS

To complement the description that will be made below and in order to help to better understand the features of the invention according to a preferred practical embodiment thereof, a set of drawings is attached as an integral part of said description in which the following is depicted in a non-limiting illustrative manner:
Figure 1 shows a rear perspective view of a loading base for facial protection devices made according to the object of the present invention, with the corresponding facial protection device arranged thereon.
Figure 2 shows a front perspective view of a loading base for facial protection devices made according to the object of the present invention, with the corresponding facial protection device arranged thereon.
Figure 3 shows the perspective view of a detail of the mechanism for lifting the disinfection bridge, with said bridge being in an inoperative situation in the figure.
Figure 4 shows a view similar to that of Figure 2, but in which the bridge is in an intermediate working situation.
Figure 5 shows the exploded view of a detail of the disinfection bridge.

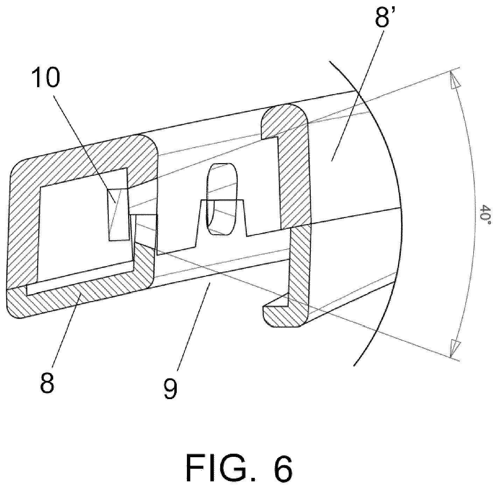

Lastly, Figure 6 shows the section view of a detail according to an imaginary vertical plane of section of the disinfection bridge.

### PREFERRED EMBODIMENT OF THE INVENTION

In view of the mentioned figures, it can be seen how the loading base for facial protection devices is made up of a main housing (1) inside which there are established the electronics for providing electric supply through a network connection cable (2) for recharging the battery of a facial protection device having a visor provided with a screen (4) associated with a housing (5), having in the front area thereof a LED lamp and a camera, including a microphone, and swinging elements to facilitate moving the visor with respect to the support forming the device, as well as an internal battery for powering the electronic equipment, said battery having an electrical connector established on the lower area of the housing (5) at the level of the support arms (6) which project from the loading base, one of which will include a complementary electrical connector for powering said battery.

The invention focuses on the fact that the base incorporates a retractable bridge (7) that can move vertically which, in an inoperative situation, is flush with the main housing (1) of the base, said bridge (7) having a structure in which two parallel arches (8-8') are defined determining an arched slot (9), according to the curvature and horizontal section of the transparent visor or screen (4) of the facial protection device, with said slot being arranged facing the screen and below same.

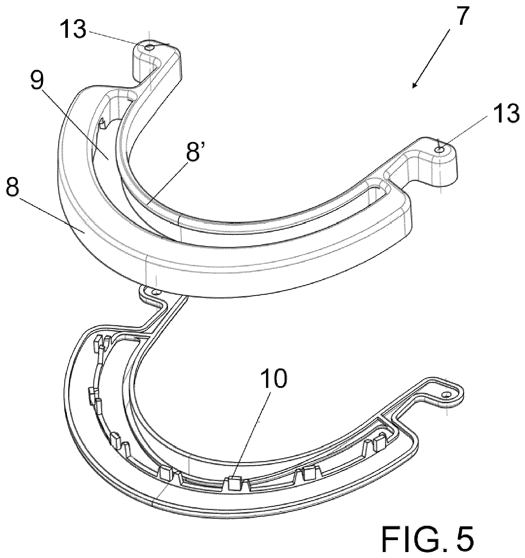

As can be seen in Figures 5 and 6, this bridge will incorporate inside its arches (8-8') a series of UV LEDs (10) intended for disinfecting both faces of the screen (4) of the facial protection device.

These UV LEDs (10) have a spectrum ranging from 200 nm to the 280 nm known as ultraviolet "UV-C" spectrum.

The bridge (7) and accordingly the arches (8-8') will be obtained in metal made by injection or machining of aluminum-Zamac or thermo-technical plastic.

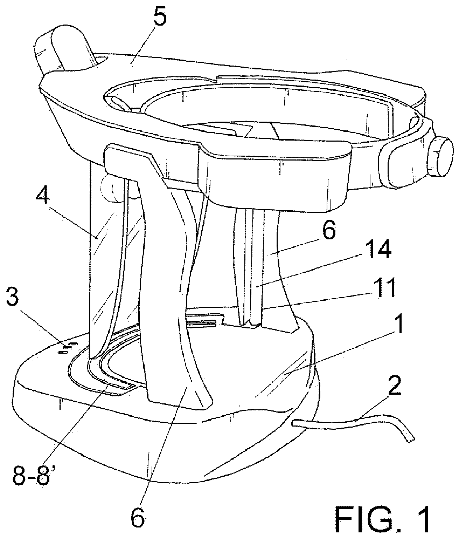

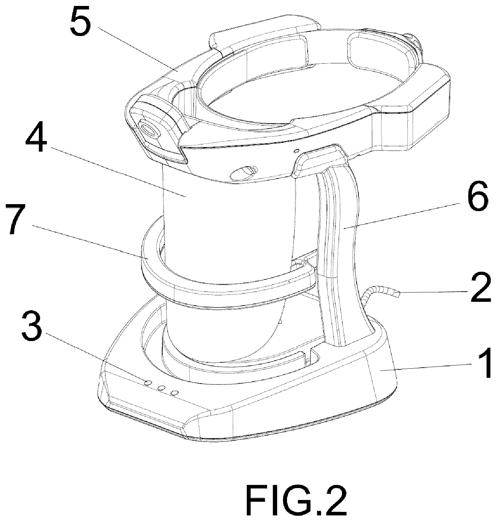

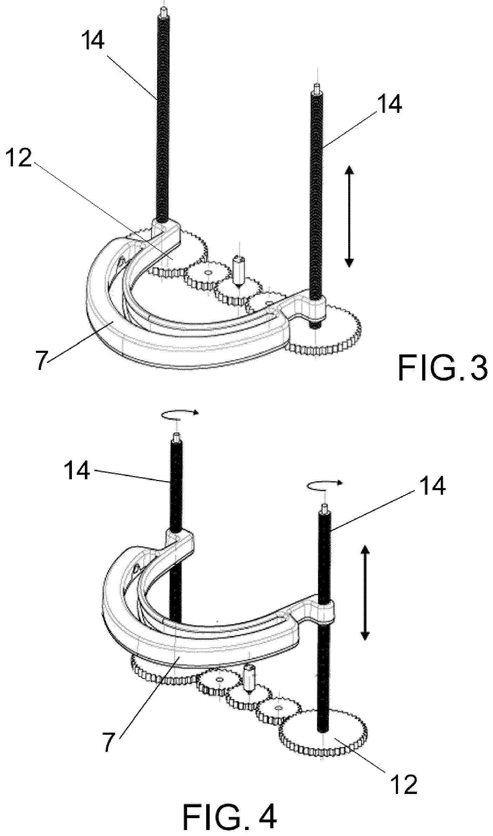

As can be seen in Figures 1, 2, 3 and 4, the bridge (7) can move vertically from a pair of vertical augers (14) provided in slots (11) made on the support arms (6) which determine the support on which the facial protection device rests.

These vertical augers (14) rotate in a synchronized manner by being linked to the ends of the bridge (7) through the corresponding threading, being actuated through a transmission (12) linked to a DC electric motor or control of paso in tensions from 3 to 230 V.

UV-C LED technology modules based on AlGaN and Flip chip, which are techniques for manufacturing devices of this type, will be arranged integrally inside the arches (8-8'). These will be housed in a flexible or rigid aluminum or copper substrate support (MPCB or PCB - printed circuit board), whereas the electronics for controlling this module (in constant current or voltage) as well as the movement of the arcs (8-8') will be arranged in the plate or plates housed in the charging base.

This movement will result in this light spectrum striking the screen (4) and will end up disinfecting the surfaces that are usually in contact with the patient or the professional. This radiation is particularly effective in breaking DNA/RNA bonds of the organisms to be eliminated; the photons will break these bonds in bacteria, fungi, viruses and pathogens in general, causing microbiological inactivation.

The power may vary to reduce the exposure time, since the higher the power, the shorter the exposure time. The facial protection device will have means for adjusting the power and exposure time through an APP in which more or less exposure will be provided depending on the use it has had.

For the pathogen neutralization or disinfection process to be safe for daily use by healthcare personnel, surgeons, orthodontists, veterinarians or any professional, the loading base incorporates an electronic safety system. The UVC radiation must always be below the limits of < 0.001 W/m2 established by the occupational safety regulations. This is achieved by placing one or more Doppler tracing devices at a frequency of 60 GHz, this search for bodies will be carried out in 360 degrees constant, i.e., it will always rotate during the disinfection time, this will provide the apparatus with absolute safety in any environment. The sensor device will be completely autonomous and its antenna will also be integrated into the base. The beam opening will be equal to or > 45º and the detection distance will also be selectable through the APP.

Returning again to Figure 1, note that the base will incorporate an interface with light indicators (3) for indicating the state of the device (fully charged/charging/being disinfected, etc.).

## Claims

1. A loading base for facial protection devices comprising a main housing (1) having support arms (6) on which a facial protection device with a screen (4) rests, **characterized in that** the loading base incorporates a bridge (7) that can move vertically on the support arms (6), wherein the bridge (7) has a structure in which two parallel arches (8-8') are defined determining an arched slot (9), said bridge (7) incorporating inside its arches (8-8') a plurality of UV LEDs (10) for disinfecting both surfaces of the screen (4) of the facial protection device.

2. The loading base for facial protection devices according to claim 1, **characterized in that** the bridge (7) can move vertically from a pair of vertical augers (14) provided in slots (11) made on the support arms (6).

3. The loading base for facial protection devices according to claim 2, **characterized in that** the ends of the bridge (7) incorporate perforations (13) through which the bridge (7) is linked to the vertical augers (14), the rotation of the vertical augers (14) being actuated by means of a transmission (12) linked to an electric motor.

4. The loading base for facial protection devices according to claim 1, **characterized in that** it includes at least one proximity sensor for detecting the presence of people in the vicinities of the loading base acting as a trigger for stopping/restarting same in order to control the switching on and off of the plurality of UV LEDs (10).

5. The loading base for facial protection devices according to claim 1, **characterized in that** it includes means for adjusting power and exposure time which are adjustable through a computer application.

6. The loading base for facial protection devices according to claim 1, **characterized in that** it includes an interface with light indicators (3) for indicating the state of the device.

7. The loading base for facial protection devices according to claim 1, **characterized in that** the bridge (7) is retractable in an inoperative situation, in which it is flush with the main housing (1) of the base.

## Patentansprüche

1. Ladebasis für Gesichtsschutzvorrichtungen umfassend ein Hauptgehäuse (1), welches Stützarme (6) aufweist, auf welchen eine Gesichtsschutzvorrichtung mit einer Abschirmung (4) aufliegt, **dadurch gekennzeichnet, dass** die Ladebasis eine Brücke (7) enthält, welche sich vertikal auf den Stützarmen (6) bewegen kann, wobei die Brücke (7) eine Struktur aufweist, in welcher zwei parallele Bögen (8-8') unter Bestimmung einer Bogennut (9) definiert sind, wobei die genannte Brücke (7) innerhalb deren Bögen (8-8') ein Vielzahl von UV-LEDs (10) zum Desinfizieren der beiden Oberflächen der Abschirmung (4) der Gesichtsschutzvorrichtung enthält.

2. Ladebasis für Gesichtsschutzvorrichtungen nach Anspruch 1, **dadurch gekennzeichnet, dass** sich die Brücke (7) vertikal von einem Paar von vertikalen Schnecken (14) aus, welche in auf den Stützarmen (6) hergestellten Nuten (11) vorgesehen sind, bewegen kann.

3. Ladebasis für Gesichtsschutzvorrichtungen nach Anspruch 2, **dadurch gekennzeichnet, dass** die Enden der Brücke (7) Bohrungen (13) enthalten, durch welche die Brücke (7) mit den vertikalen Schnecken (14) verbunden ist, wobei die Rotation der vertikalen Schnecken (14) mittels eines Getriebes (12) betätigt wird, welches mit einem Elektromotor verbunden ist.

4. Ladebasis für Gesichtsschutzvorrichtungen nach Anspruch 1, **dadurch gekennzeichnet, dass** sie mindestens einen Näherungssensor zum Detektieren der Anwesenheit von Personen in der nahen Umgebung der Ladebasis beinhaltet, was als Auslöser zum Stoppen/Neustarten derselben wirkt, um das Ein- und Ausschalten der Vielzahl von UV-LEDs (10) zu steuern.

5. Ladebasis für Gesichtsschutzvorrichtungen nach Anspruch 1, **dadurch gekennzeichnet, dass** sie Mittel zum Einstellen der Leistung und der Einwirkungszeit beinhaltet, welche über eine Computeranwendung einstellbar sind.

6. Ladebasis für Gesichtsschutzvorrichtungen nach Anspruch 1, **dadurch gekennzeichnet, dass** sie eine Schnittstelle mit Lichtanzeigern (3) zum Anzeigen des Zustands der Vorrichtung beinhaltet.

7. Ladebasis für Gesichtsschutzvorrichtungen nach Anspruch 1, **dadurch gekennzeichnet, dass** die Brücke (7) in eine betriebsunfähige Situation einfahrbar ist, in welcher sie mit dem Hauptgehäuse (1) der Basis bündig ist.

## Revendications

1. Base de charge pour dispositifs de protection faciale comprenant un logement principal (1) ayant des bras de support (6) sur lequel s'appuie un dispositif de protection avec un écran (4), **caractérisée en ce que** la base de charge comporte un pont (7) qui peut se déplacer verticalement sur les bras de support (6), dans laquelle le pont (7) présente une structure dans laquelle deux arcs parallèles (8-8') sont définis déterminant une fente arquée (9), ledit pont (7) comportant à l'intérieur de ses arcs (8-8') une pluralité de LED UV (10) pour désinfecter les deux surfaces de l'écran (4) du dispositif de protection faciale.

2. Base de charge pour dispositifs de protection faciale selon la revendication 1, **caractérisée en ce que** le pont (7) peut se déplacer verticalement à partir d'une paire de vis sans fin verticales (14) prévues dans des fentes (11) réalisées sur les bras de support (6).

3. Base de charge pour dispositifs de protection faciale selon la revendication 2, **caractérisée en ce que** les extrémités du pont (7) comportent des perforations (13) à travers lesquelles le pont (7) est relié aux vis sans fin verticales (14), la rotation des vis sans fin verticales (14) étant actionnée par le biais d'une transmission (12) reliée à un moteur électrique.

4. Base de charge pour dispositifs de protection faciale selon la revendication 1, **caractérisée en ce qu'**elle comporte au moins un capteur de proximité pour détecter la présence de personnes à proximité de la base de charge agissant en tant que déclencheur pour arrêter/redémarrer celle-ci afin de contrôler l'allumage et l'extinction de la pluralité de LED UV (10).

5. Base de charge pour dispositifs de protection faciale selon la revendication 1, **caractérisée en ce qu'**elle comporte des moyens pour régler la puissance et le temps d'exposition qui peuvent être réglés par le biais d'une application informatique.

6. Base de charge pour dispositifs de protection faciale selon la revendication 1, **caractérisée en ce qu'**elle comporte une interface avec des indicateurs lumineux (3) pour indiquer l'état du dispositif.

7. Base de charge pour dispositifs de protection faciale selon la revendication 1, **caractérisée en ce que** le pont (7) est escamotable dans une situation inopérante, dans laquelle il est aligné avec le logement principal (1) de la base.
